(19) Europäisches Patentamt
European Patent Office
Office européen des brevets

(11) Publication number: 0 010 401
B1

(12) **EUROPEAN PATENT SPECIFICATION**

(45) Date of publication of patent specification: 05.05.82

(21) Application number: 79302167.6

(22) Date of filing: 10.10.79

(51) Int. Cl.[3]: C 07 C 161/04, C 07 D 333/22, C 07 D 213/53, A 01 N 47/46

(54) **Novel (1-substituted) cycloalkylmethyl-isothiocyanates, a process for their preparation, compositions comprising them, and their use as pesticides.**

(30) Priority: 13.10.78 US 950968
16.05.79 US 39613

(43) Date of publication of application:
30.04.80 Bulletin 80/9

(45) Publication of the grant of the patent:
05.05.82 Bulletin 82/18

(84) Designated Contracting States:
AT BE CH DE FR GB IT LU NL SE

(56) References cited:
DE - A - 2 157 048
US - A - 3 031 486
US - A - 3 367 957

(73) Proprietor: JANSSEN PHARMACEUTICA N.V.
Turnhoutsebaan 30
B-2340 Beerse (BE)

(72) Inventor: van der Aa, Marcel
Merellaan 46
B-2350 Vosselaar (BE)
Inventor: Stokbroekx, Raymond
Rode Kruisstraat 13
D-2340 Beerse (BE)

(74) Representative: Jones, Alan John et al,
CARPMAELS & RANSFORD 43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 010 401 B1

Courier Press, Leamington Spa, England.

# Novel (1-substituted) Cycloalkylmethyl-isothiocyanates, a process for their preparation, compositions comprising them, and their use as pesticides

## Background of the invention:

In Brit. Pat. No. 892,790 and in U.S. Pat. No. 3,367,957 there are described respectively the compounds methyl isothiocyanate and cyclopropylmethyl isothiocyanate both as nematocides.

The present invention relates to a novel class of biocidal (1-substituted) cycloalkylmethyl-isothiocyanates which differ from the aforementioned cyclopropylmethyl isothiocyanate by the presence of an aryl or heterocyclic radical in the 1-position of the cycloalkane ring.

## Description of the preferred embodiments:

This invention is concerned with novel (1-aryl (or heterocyclic) cycloalkylmethyl) isothiocyanates which may structurally be represented by the formula (I)

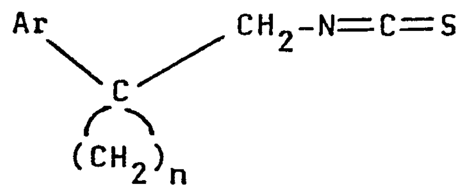

(I)

wherein

Ar is thienyl, halothienyl, naphthalenyl, pyridinyl, phenyl or substituted phenyl, said substituted phenyl having from 1 to 3 substituents, each independently selected from lower alkyl, lower alkyloxy, lower alkylthio, halo, amino, nitro, cyano and trifluoromethyl; and n is an integer of from 2 to 5 inclusive.

In the foregoing and in the following definitions, the term "halo" is generic to fluoro, chloro, bromo and iodo; the term "lower alkyl" is meant to include straight and branched hydrocarbon radicals, having from 1 to 6 carbon atoms, such as, for example, methyl, ethyl, propyl, butyl, pentyl, hexyl, 1-methylethyl, 1,1-dimethylethyl and the like.

The compounds of Formula (I) may be prepared by the application of methodologies known in the art, as described, for example, in Saul Patai Ed. "The chemistry of cyanates and their thioderivatives" John Wiley & Sons Chichester-New York-Brisbane-Toronto (1977) p. 1013—1053. One method of preparing the compounds (I) is by the reaction of the amine (II) with carbon disulfide (III) to obtain a dithiocarbamate of formula (IV) and subsequently converting the latter into the desired isothiocyanate (I) by the reaction of (IV) with a decomposing agent consisting of a lower alkyl chloroformate (V), preferably ethyl chloroformate. The foregoing reactions are illustrated as follows:

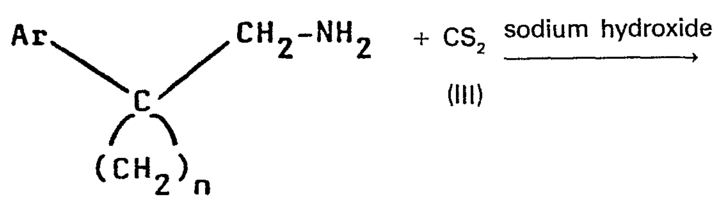


(III)

(II)

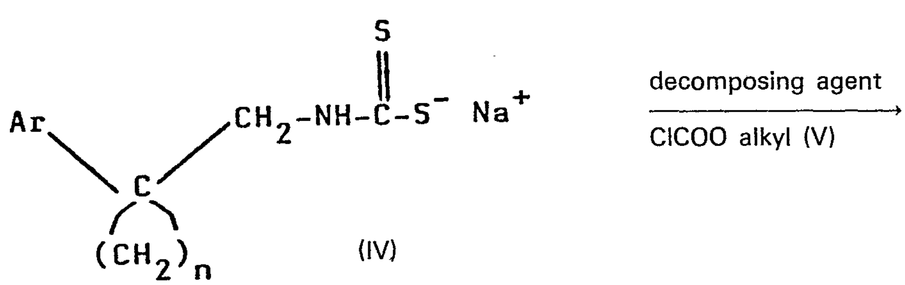


(IV)

(I)

Said reaction of the amine (II) with carbon disulfide (III) is conveniently carried out in an appropriate reaction-inert solvent such as, for example, water; a lower alcohol, e.g. methanol; an alifatic-, alicyclic- or aromatic hydrocarbon, e.g. hexane, cyclohexane or benzene; a halogenated hydrocarbon e.g. trichloromethane; an alifatic- or alicyclic ether, e.g. 1,1'-oxybispropane or 1,4-dioxane; and the like; and in the presence of sodium hydroxide. Preferably the reaction-temperature is kept below room temperature and, most preferably between 0°C and 10°C.

The decomposition-reaction of the dithiocarbamate (IV) by its reaction with the lower alkyl

chloroformate (V) is suitably conducted in an appropriate reaction-inert solvent, e.g. one of the solvents described hereabove for the reaction of (II) with (III). Bases such as alkaline metal- or earth alkaline metal hydroxide, e.g. potassium hydroxide, or tertiary amines, e.g. N,N-diethylethanamine are taught to have a catalytic effect on the decomposition of the intermediately formed carbalkoxydithiocarbamate (VI). (See e.g. J. Am. Chem. Soc. *83,* 2532—2536 (1961)). Somewhat elevated temperatures may be used to enhance the reaction-rate.

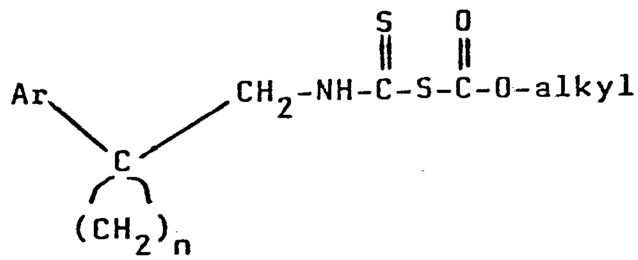

(VI)

The amines of formula (II), used as starting materials herein, are generally known and they may all be prepared following methods described in the literature for the preparation of such known or similar compounds.

The isothiocyanates of formula (I) possess valuable biocidal properties and as such they can be used in a wide variety of circumstances.

More particularly, the subject compounds have fungicidal, bactericidal and insecticidal properties, and as a result they can be used to combat any unwanted growth of fungi or bacteria or to kill insects.

The compounds of formula (I) are especially useful as preservatives of organic materials which are susceptible to attack by noxious microorganisms and insect. They can be used, for example, for the preservation of technical and agricultural products, such as, for example, wood, fibers, textiles, jute, cork, paper, dyes, etc., and as disinfectants in veterinary medium and human hygiene. The subject compounds are also active against marine organisms and as such they can be used as antifouling agents.

The useful biocidal properties of the compounds of formula (I) are clearly illustrated in the following experiments.

A. Insecticidal activity against the house-fly (Musca domestica)

Test solutions are prepared by dissolving the test-compounds in 2-propanone at a concentration of 2.5% and further diluting the thus obtained stock-solution with water to obtain final concentrations of the active ingredient of respectively 0.02, 0.01, 0.005 and 0.0025%. 1 ml of the test solution is poured onto a filter paper of 11 cm diameter. The filter paper is covered by a petri-dish of 11 cm diameter and 10 flies are confined in the space between the filter paper and the petri-dish. Insecticidal activity of the test compound is assessed 1, 4 and 24 hours thereafter by counting the number of flies that are respectively alive, knocked-down or dead.

The useful insecticidal properties of the compounds of formula (I) are clearly illustrated by the results presented in the following Table I.

TABLE I: Efficacy of compounds (I) against house-fly.

| Compound | Conc. of act. ingredient in test solution | Number of flies alive (L), knocked-down (KD) and dead (D) | | | | | | | | |
|---|---|---|---|---|---|---|---|---|---|---|
| | | After 1 hour | | | After 4 hours | | | After 24 hours | | |
| | | L | KD | D | L | KD | D | L | KD | D |
| 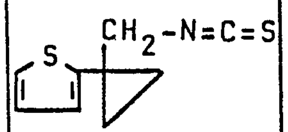 | 0.02% | 8 | 2 | 0 | 0 | 2 | 8 | 0 | 0 | 10 |
| | 0.01% | 9 | 1 | 0 | 3 | 3 | 4 | 0 | 0 | 10 |
| | 0.005% | 8 | 2 | 0 | 6 | 3 | 1 | 0 | 0 | 10 |
| | 0.0025% | 10 | 0 | 0 | 8 | 1 | 1 | 2 | 2 | 6 |
| 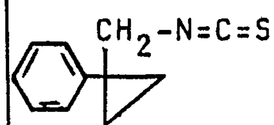 | 0.02% | 8 | 2 | 0 | 4 | 0 | 6 | 0 | 0 | 10 |
| | 0.01% | 8 | 2 | 0 | 3 | 3 | 4 | 0 | 0 | 10 |
| | 0.005% | 9 | 1 | 0 | 3 | 5 | 2 | 0 | 0 | 10 |
| | 0.0025% | 10 | 0 | 0 | 10 | 0 | 0 | 4 | 1 | 5 |

B. Activity against wood-attacking fungi.

The tests are performed in petri-dishes of 50 mm diameter using a solid malt-agar medium. The test compound is incorporated into the medium by mixing the latter, before solidification, with a solution of the test compound in 50% ethanol to obtain concentrations of the active ingredient of from 0.1 to 100 ppm (parts per million). After solidification of the medium inoculation is performed by applying a piece of mycelium to the center of each petri-dish. Antifungal activity is evaluated 7 and 14 days after inoculation by estimating the surface of each petri-dish which is covered by mycelium of the fungus. The results, represented in the following Table II, are expressed according to the following score system:

score 0 = no growth

score 1 = $\leqslant$ 25% growth as compared to untreated control.

score 2 = 25 to 50% growth as compared to untreated control.

score 3 = 50 to 75% growth as compared to untreated control.

score 4 = $>$ 75% growth as compared to untreated control.

For each test 2 scores are given, representing respectively the evaluations made after 7 and 14 days.

TABLE II: Activity of compounds (I) against wood-attacking fungi.

| Compound | Concentration of act. ingredient in ppm | Anti-fungal scores | | | | | | | |
|---|---|---|---|---|---|---|---|---|---|
| | | Coniophora cerebella | | Coriolus versicolor | | Poria monticola | | Pullularis pullulans | |
| | | 7d. | 14d. | 7d. | 14d. | 7d. | 14d. | 7d. | 14d. |
| 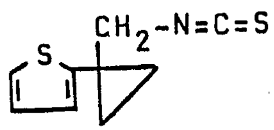 | 100 | 0 | 0 | 0 | 0 | 0 | 0 | 1 | 3 |
| | 10 | 0 | 0 | 4 | 4 | 0 | 0 | 4 | 4 |
| | 1 | 3 | 4 | 4 | 4 | 4 | 4 | 4 | 4 |
| | 0.1 | 4 | 4 | 4 | 4 | 4 | 4 | 4 | 4 |
| 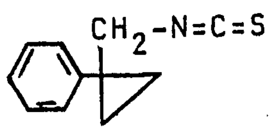 | 100 | 0 | 0 | 0 | 0 | 0 | 0 | 2 | 4 |
| | 10 | 0 | 0 | 0 | 0 | 0 | 0 | 4 | 4 |
| | 1 | 3 | 4 | 4 | 4 | 4 | 4 | 4 | 4 |
| | 0.1 | – | – | – | – | – | – | – | – |

In view of the aforementioned biocidal activities of the compounds of the invention this invention further provides valuable compositions comprising at least one of the isothiocyanates of formula (I) as an active ingredient, if desired, in admixture with other biocidal active agents, in a solvent or a solid, a semi-solid or a liquid diluent or a carrier.

The active agents can be used in suitable solvents or diluents in the form of emulsions, suspensions, dispersions or ointments, on suitable solid or semi-solid carrier substances, in ordinary or synthetic soaps, detergents or dispersion media.

Solid carrier substances which are suitable for the preparation of compositions in powder form include various inert, porous and pulverous distributing agents of inorganic or organic nature such as, for example, tricalcium phosphate or calcium carbonate, in the form of prepared chalk or ground limestone, kaolin, bole, bentonite, talcum, silic acid or boric acid; powdered cork, sawdust, and other fine pulverous materials of vegetable origin are also carrier substances.

The isothiocyanates are mixed with these carrier substances, for example by being ground therewith; alternatively, the inert carrier substance is impregnated with a solution of the active component in a readily volatile solvent and the solvent is thereafter eliminated by heating or by filtering with suction at reduced pressure. By adding wetting and/or dispersing agents, pulverous preparations can also be made readily wettable with water, so that suspensions are obtained.

Inert solvents used for the production of liquid preparations should preferably not be readily inflammable and as far as possible non-toxic to warm-blooded animals or plants in the relevant surroundings. Solvents suitable for this purpose are high-boiling oils, for example, of vegetable origin, and lower-boiling solvents, such as, for example, isopropanol, dimethylsulfoxide, hydrogenated naphthalenes and alkylated naphthalenes. It is of course, also possible to use mixtures of solvents. Solutions can be prepared in the usual way, if necessary, with assistance of solution promoters. Other liquid forms which can be used consist of emulsions or suspensions of the active compound in water or suitable inert solvents, or also concentrates for preparing such emulsions, which can be directly adjusted to the required concentrations. For this purpose, the isothiocyanates are, for example, mixed with a dispersing or emulsifying agent. The active component can also be dissolved or dispersed in a suitable inert solvent and mixed simultaneously or subsequently with a dispersing or emulsifying agent.

It is also possible to use semi-solid carrier substances of a cream ointment, paste or waxlike nature, into which the isothiocyanates can be incorporated, if necessary, with the aid of solution promoters and/or emulsifiers. Vaseline and other cream bases are examples of semi-solid carrier substances.

Furthermore, it is possible for the isothiocyanates to be used in the form of aerosols. For this purpose, the active component is dissolved or dispersed, if necessary, with the aid of suitable inert solvents as carrier liquids, such as difluorodichloromethane, which at atmospheric pressure boils at a temperature lower than room temperature, or in other volatile solvents. In this way, solutions under pressure are obtained which, when sprayed, yield aerosols which are particularly suitable for controlling or combating fungi and bacteria, e.g. in closed chambers and storage rooms, and for application to vegetation for eradicating or for preventing infection by fungi or bacteria.

The isothiocyanates and the compositions thereof can be applied by conventional methods. For example a growth of microorganisms or a material to be treated or to be protected against attack by microorganisms can be treated with the isothiocyanates and the compositions thereof by dusting, sprinkling, spraying, brushing, dipping, smearing, impregnating or other suitable means.

When the isothiocyanates are employed in combination with suitable carriers, e.g. in solution, suspension, dust, powder, ointment, emulsion and the like forms, a high activity over a very high range of dilution is observed. For example, concentrations of the active ingredient ranging from 0.1 to 10 percent by weight, based on the weight of composition employed, have been found effective in combating microorganisms. Higher concentrations may naturally also be employed as warranted by the particular situation.

The compounds of formula (I) may be applied alone or optionally together with other biocidal substances, e.g., bactericides, fungicides, insecticides etc. When used in the preservation of organic material, particularly wood, it has been found advantageous to combine the isothiocyanates of formula (I) with antimicrobial imidazole and triazole derivatives such as for example, 1-[2-(2,4-dichlorophenyl)-2-(2-propenyloxy)ethyl]-1H-imidazole, 1-[2-(2,4-dichlorophenyl)-1,3-dioxolan-2-ylmethyl]-1H-1,2,4-triazole, 1-[2-(2,4-dichlorophenyl)-4-ethyl-1,3-dioxolan-2-ylmethyl]-1H-1,2,4-triazole, 1-(4-chlorophenoxy)-1-(1H-1,2,4-triazol-1-yl)-3,3-dimethylbutan-2-one and the like.

The following examples are intended to illustrate and not to limit the scope of the present invention.

### Example I

To a solution of 6.5 parts of sodium hydroxide in 120 parts of water are added 12.5 parts of carbon disulfide, while cooling (±10°C). Then there are added dropwise 23 parts of 1-phenylcyclopropylmethylamine (exothermic reaction). The whole is stirred for 30 minutes at room temperature. At a temperature of 30°C, there are added dropwise 17 parts of ethyl chloroformate (exothermic reaction: cooling is necessary). Upon completion, the mixture is stirred for 3 hours at 50°C. The product is

extracted with benzene. The organic layer is dried and evaporated. The residue is distilled twice, yielding 18 parts of (1-phenylcyclopropylmethyl) isothiocyanate; bp. 150°C at 10 mm pressure; $n_d^{20}$: 1.5831; $d_{20}^{20}$: 1.0917.

Following the same procedure and using equivalent amounts of the appropriate starting materials there are also prepared:

[1-(4-chlorophenyl)cyclopropylmethyl]isothiocyanate; bp. 131—133°C at 0.4 mm pressure; $n_D^{20}$: 1.5936; $d_{20}^{20}$: 1.2022;

[1-(4-methoxyphenyl)cyclopropylmethyl]isothiocyanate; bp. 120°C at 0.1 mm pressure; $n_D^{20}$: 1.5811; $d_{20}^{20}$: 1.1286; and

[1-(4-fluorophenyl)cyclopropylmethyl]isothiocyanate; bp. 152—153°C at 10 mm pressure; $n_D^{20}$: 1.5648; $d_{20}^{20}$ 1.1674.

Example II

A solution of 20 parts of sodium hydroxide and 300 parts of water is cooled to ±10°C and there are added successively 41.8 parts of carbon disulfide and then dropwise 95 parts of 1-phenylcyclopentylmethylamine (exothermic reaction). Upon completion, the cooling-bath is removed and the whole is stirred for 30 minutes at room temperature. Then there are added dropwise at 30°C, 54.25 parts of ethyl chloroformate (exothermic reaction: cooling is necessary). The whole is stirred for 3 hours at 50°C. The reaction mixture is cooled and the product is extracted with benzene. The extract is dried, filtered and evaporated. The oily residue is distilled, yielding 86 parts of (1-phenylcyclopentylmethyl) isothiocyanate; bp. 125°C at 0.2 mm pressure; $n_D^{20}$: 1.5848; $d_{20}^{20}$: 1.0910.

Following the same procedure and using equivalent amounts of the appropriate starting materials there are also prepared:

[1-(4-fluorophenyl)cyclopentylmethyl]isothiocyanate; bp. 120°C at 0.2 mm pressure;

[1-(4-chlorophenyl)cyclopentylmethyl] isothiocyanate; mp. 48°C; and

[1-(4-methoxyphenyl)cyclopentylmethyl] isothiocyanate; mp. 69.5°C.

Example III

A solution of 11 parts of potassium hydroxide in 200 parts of water is cooled to about 10°C and there are added successively 22.8 parts of carbon disulfide and then dropwise 42 parts of 1-(2-thienyl)cyclopropylmethylamine (exothermic reaction). Upon completion, the whole is stirred for 30 minutes at room temperature. Then there are added at 30°C 29.8 parts of ethyl chloroformate (exothermic reaction: cooling is necessary). The whole is stirred for 3 hours while heating at 50°C. The reaction mixture is cooled and the product is extracted with benzene. The extract is dried, filtered and evaporated. The oily residue is distilled twice, yielding 40 parts of [1-(2-thienyl)cyclopropylmethyl]isothiocyanate; bp. 140—150°C at 10 mm pressure; $n_D^{20}$: 1.6008 $d_{20}^{20}$: 1.1911.

Example IV

A solution of 21.2 parts of sodium hydroxide in 500 parts of water is cooled to 10°C and there is added successively 45.6 parts of carbon disulfide and then dropwise 95.83 of 1-(2-thienyl)-cyclopentanemethylamine (exothermic reaction). After the addition is complete, the whole is stirred for 30 minutes at room temperature. Then there are added 57.5 parts of ethyl chloroformate at 30°C (exothermic reaction: cooling is necessary). The whole is stirred for 3 hours at 50°C and further stirred overnight without heating. The product is extracted with benzene. The extract is dried, filtered and evaporated. The residue is poured into 350 parts of water and the whole is heated for 3 hours at 80—90°C. The mixture is cooled and the product is extracted with dichloromethane. The extract is dried, filtered and evaporated. The oily residue is distilled twice, yielding 88 parts of [1-(2-thienyl)cyclopentylmethyl]isothiocyanate; bp. 110°C at 0.1 mm pressure; $n_D^{20}$: 1.5960; $d_{20}^{20}$: 1.1743.

Example V

Following the procedure described in Example II and using equivalent amounts of the appropriate starting materials there are also prepared:

[1-(4-methylphenyl)cyclopentylmethyl]isothiocyanate;
[1-(2-cyano-3-ethylthiophenyl)cyclopentylmethyl]isothiocyanate;
[1-(2-amino-4-nitrophenyl)cyclopentylmethyl]isothiocyanate;
[1-(4-trifluoromethylphenyl)cyclopentylmethyl]isothiocyanate;
[1-(4-chloro-3-thienyl)cyclopentylmethyl]isothiocyanate;
[1-(2-naphthalenyl)cyclopentylmethyl]isothiocyanate;
[1-(2-pyridinyl)cyclopentylmethyl]isothiocyanate;
(1-phenylcyclobutylmethyl)isothiocyanate;
[1-(4-methylphenyl)cyclobutylmethyl]isothiocyanate;
[1-(3-ethoxyphenyl)cyclobutylmethyl]isothiocyanate;
[1-(3-methylthiophenyl)cyclobutylmethyl]isothiocyanate;
[1-(4-cyano-2,6-dibromophenyl)cyclobutylmethyl]isothiocyanate;

[1-(2-amino-4-nitrophenyl)cyclobutylmethyl]isothiocyanate;
[1-(2-thienyl)cyclobutylmethyl]isothiocyanate;
[1-(4-chloro-2-thienyl)cyclobutylmethyl]isothiocyanate;
[1-(2-naphthalenyl)cyclobutylmethyl]isothiocyanate;
[1-(3-pyridinyl)cyclobutylmethyl]isothiocyanate;
[1-(2-ethylphenyl)cyclohexylmethyl]isothiocyanate;
[1-(2-chloro-3-methylthiophenyl)cyclohexylmethyl]isothiocyanate;
[1-(3-propyloxyphenyl)cyclohexylmethyl]isothiocyanate;
[1-(2-amino-4-nitrophenyl)cyclohexylmethyl]isothiocyanate;
[1-(2-cyano-4-trifluoromethylphenyl)cyclohexylmethyl]isothiocyanate;
[1-(5-bromo-3-thienyl)cyclohexylmethyl]isothiocyanate;
[1-(1-naphthalenyl)cyclohexylmethyl]isothiocyanate;
[1-(1-pyridinyl)cyclohexylmethyl]isothiocyanate; and
[1-phenylcyclohexylmethyl)isothiocyanate.

Example VI

Following the procedure described in Example I and using equivalent amounts of the appropriate starting materials there are also prepared:
[1-(4-propylphenyl)cyclopropylmethyl]isothiocyanate;
[1-(2-amino-5-methylphenyl)cyclopropylmethyl]isothiocyanate;
[1-(4-cyano-2-nitrophenyl)cyclopropylmethyl]isothiocyanate;
[1-(3-trifluoromethylphenyl)cyclopropylmethyl]isothiocyanate;
[1-(5-bromo-2-thienyl)cyclopropylmethyl]isothiocyanate;
[1-(1-naphthalenyl)cyclopropylmethyl]isothiocyanate; and
[1-(4-pyridinyl)cyclopropylmethyl]isothiocyanate.

**Claims for the Contracting States: BE, CH, DE, FR, GB, IT, LV, NL, SE**

1. A (1-aryl (or heterocyclic) cycloalkylmethyl) isothiocyanate having the formula

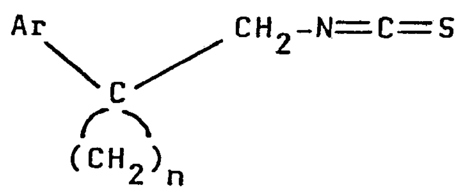

wherein

Ar is thienyl, halothienyl, naphthalenyl, pyridinyl, phenyl or substituted phenyl, said substituted phenyl having from 1 to 3 substituents, each independently selected from $C_1$—$C_6$ alkyl, $C_1$—$C_6$ alkyloxy, $C_1$—$C_6$ alkylthio, halo, amino, nitro, cyano and trifluoromethyl; and

n is an integer of from 2 to 5 inclusive.

2. (1-Phenylcyclopropylmethyl)isothiocyanate.

3. [1-(2-Thienyl)cyclopropylmethyl]isothiocyanate.

4. A process for preparing a (1-aryl (or heterocyclic) cycloalkylmethyl)isothiocyanate having the formula

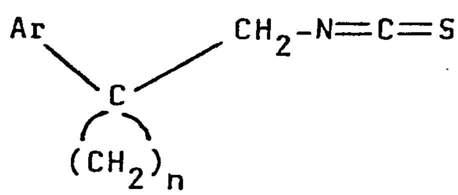

(I)

wherein

Ar is thienyl, halothienyl, naphthalenyl, pyridinyl, phenyl or substituted phenyl, said substituted phenyl having from 1 to 3 substituents, each independently selected from $C_1$—$C_6$ alkyl, $C_1$—$C_6$ alkyloxy, $C_1$—$C_6$ alkylthio, halo, amino, nitro, cyano and trifluoromethyl; and

n is an integer of from 2 to 5 inclusive; characterized by reacting a compound of the formula

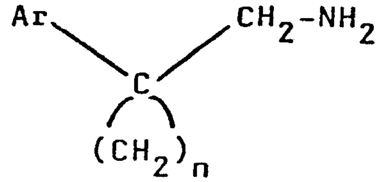

(II)

with carbon disulfide (III) in the presence of sodium hydroxide in order to prepare a compound of the formula

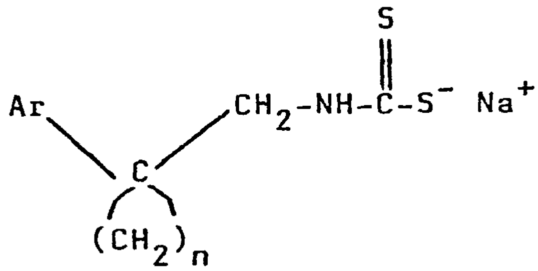

(IV)

subsequently converting the latter into the desired isothiocyanate (I) by the reaction of (IV) with a decomposing agent consisting of a $C_1$—$C_6$ alkyl chloroformate, said reaction of the amine (II) with carbon disulfide (III) being carried out in an appropriate reaction-inert solvent, the reaction-temperature being kept below room temperature, and the decomposition-reaction of the dithiocarbamate (IV) by its reaction with the $C_1$—$C_6$-alkyl chloroformate (V) being suitably conducted at elevated temperatures in an appropriate reaction-inert solvent, and a base is added to produce a catalytic effect on the decomposition of the intermediately formed carbalkoxydithiocarbamate of the formula

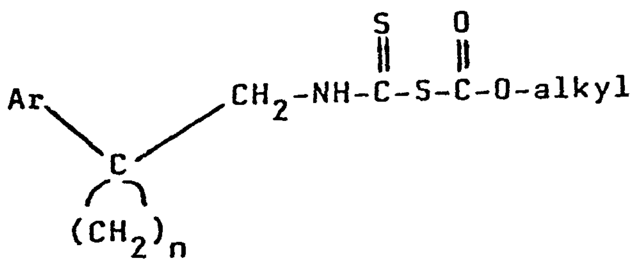

(VI)

5. A composition for combating fungi, bacteria and insects comprising an inert carrier material and, as an active ingredient, an effective amount of a (1-aryl (or heterocyclic) cycloalkylmethyl)isothiocyanate having the formula

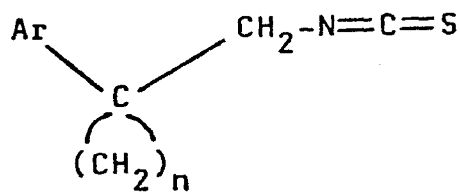

wherein

Ar is thienyl, halothienyl, naphthalenyl, pyridinyl, phenyl or substituted phenyl, said substituted phenyl having from 1 to 3 substituents, each independently selected from $C_1$—$C_6$ alkyl, $C_1$—$C_6$ alkyloxy, $C_1$—$C_6$ alkylthio, halo, amino, nitro, cyano and trifluoromethyl; and

n is an integer of from 2 to 5 inclusive.

6. A composition in accordance with Claim 5 wherein said isothiocyanate is as claimed in Claim 2 or Claim 3.

7. A compound in accordance with any one of Claims 1 to 3, or a composition in accordance with Claim 5 or Claim 6, for use as a fungicidal, bactericidal, or insecticidal agent.

**Claims for the Contracting State: AT**

1. A process for preparing a (1-aryl (or heterocyclic) cycloalkylmethyl)isothiocyanate having the formula

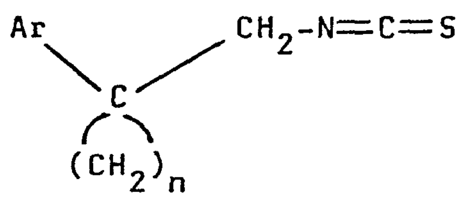

(I)

wherein

Ar is thienyl, halothienyl, naphthalenyl, pyridinyl, phenyl or substituted phenyl, said substituted phenyl having from 1 to 3 substituents, each independently selected from $C_1$—$C_6$ alkyl, $C_1$—$C_6$

alkyloxy, $C_1$—$C_6$ alkylthio, halo, amino, nitro, cyano and trifluoromethyl; and

n is an integer of from 2 to 5 inclusive; characterized by reacting a compound of the formula

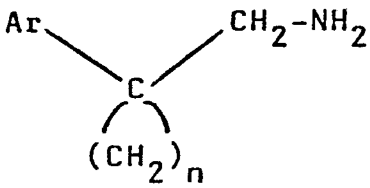

(II)

with carbon disulfide (III) in the presence of sodium hydroxide in order to prepare a compound of the formula

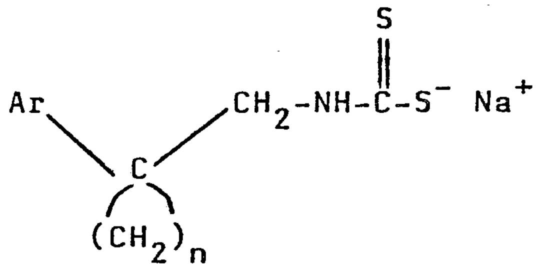

(IV)

subsequently converting the latter into the desired isothiocyanate (I) by the reaction of (IV) with a decomposing agent consisting of a $C_1$—$C_6$ alkyl chloroformate, said reaction of the amine (II) with carbon disulfide (III) being carried out in an appropriate reaction-inert solvent, the reaction-temperature being kept below room temperature, and the decomposition-reaction of the dithiocarbamate (IV) by its reaction with the $C_1$—$C_6$-alkyl chloroformate (V) being suitably conducted at elevated temperatures in an appropriate reaction-inert solvent, and a base is added to produce a catalytic effect on the decomposition of the intermediately formed carbalkoxydithiocarbamate of the formula

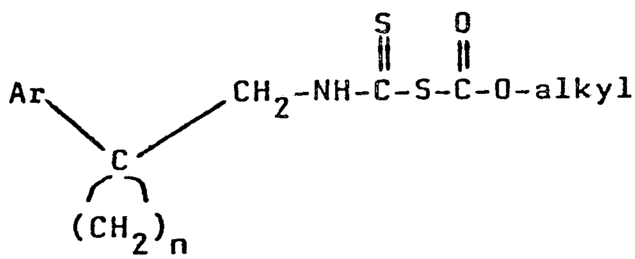

(VI)

2. A process according to Claim 1 for preparing (1-phenylcyclopropylmethyl)isothiocyanate.

3. A process according to Claim 1 for preparing [1-(2-thienyl)cyclopropylmethyl]isothiocyanate.

4. A process according to any one of Claims 1 to 3 wherein said $C_1$—$C_6$-alkyl chloroformate (V) is ethyl chloroformate.

5. A process according to any one of Claims 1 to 4 wherein said base is an alkaline metal or earth alkaline metal hydroxide.

6. A composition for combating fungi, bacteria and insects comprising an inert carrier material and, as an active ingredient, an effective amount of a (1-aryl (or heterocyclic) cycloalkylmethyl)-isothiocyanate having the formula

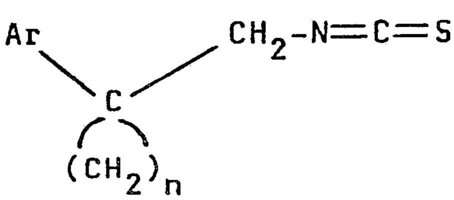

wherein

Ar is thienyl, halothienyl, naphthalenyl, pyridinyl, phenyl or substituted phenyl, said substituted phenyl having from 1 to 3 substituents, each independently selected from $C_1$—$C_6$ alkyl, $C_1$—$C_6$ alkyloxy, $C_1$—$C_6$ alkylthio, halo, amino, nitro, cyano and trifluoromethyl; and

n is an integer of from 2 to 5 inclusive.

7. A composition in accordance with Claim 6 wherein said isothiocyanate is (1-phenylcyclopropylmethyl)isothiocyanate or [1-(2-thienyl)cyclopropylmethyl]isothiocyanate.

**Revendications pour les Etats contractants: BE, CH, DE, FR, GB, IT, W, NL, SE**

1. (1-aryl(ou hétérocyclique)cycloalkylméthyl)isothiocyanate ayant pour formule

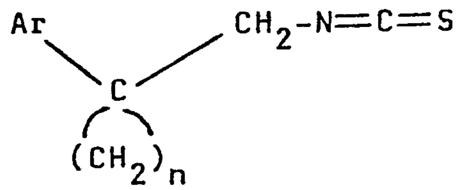

dans lequel

Ar est thiényle, halothiényle, naphtalényle, pyridinyle, phényle ou phényle substitué, ledit phényle substitué ayant de 1 à 3 substituants, chacun étant indépendamment choisi parmi un alcoyle de 1 à 6 atomes de carbone, alcoyloxy de 1 à 6 atomes de carbone, un alkylthio de 1 à 6 atomes de carbone, halo, amino, nitro, cyano et trifluorométhyle; et

n est un nombre entier de 2 à 5 inclus.

2. (1-phénylcyclopropylméthyl)isothiocyanate.

3. [1-(2-thiényl)cyclopropylméthyl]isothiocyanate.

4. Procédé de préparation d'un (1-aryl(ou hétérocyclique)cycloalkylméthyl)isothiocyanate ayant pour formule

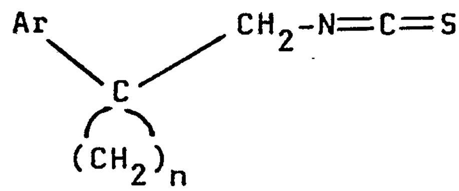

(I)

dans lequel

Ar est thiényle, halothiényle, naphtalényle, pyridinyle, phényle ou phényle substitué, ledit phényle substitué ayant de 1 à 3 substituants, chacun étant indépendamment choisi parmi un alcoyle de 1 à 6 atomes de carbone, un alcoyloxy de 1 à 6 atomes de carbone, un alkylthio de 1 à 6 atomes de carbone, halo, nitro, cyano, amino, et trifluorométhyle; et

n est un nombre entier de 2 à 5 inclus; caractérisé par la réaction d'un composé de formule

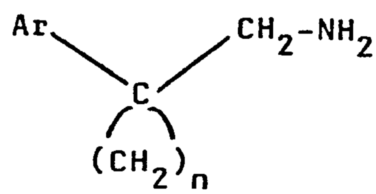

(II)

avec du disulfure de carbone (III) en présence de soude afin de préparer un composé de formule

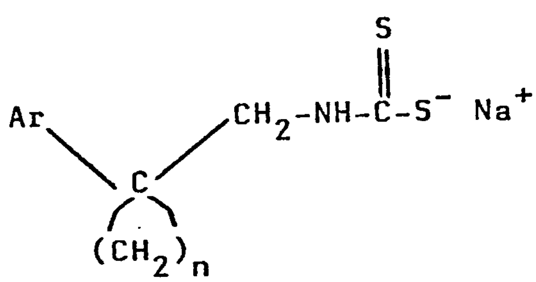

(IV)

en convertissant subséquemment ce dernier en isothiocyanate souhaité (I) par la réaction de (IV) avec un agent de décomposition consistant en un chloroformiate d'alcoyle de 1 à 6 atomes de carbone, ladite réaction de l'amine (II) avec du disulfure de carbone (III) étant effectuée dans un solvant inerte à la réaction approprié, la température de la réaction étant maintenue en dessous de la température ambiante et la réaction de décomposition du dithiocarbamate (IV) par sa réaction avec le chloroformiate d'alcoyle de 1 à 6 atomes de carbone (V) étant avantageusement entreprise à des températures élevées

dans un solvant inerte à la réaction approprié et une base est ajoutée pour produire un effet catalytique sur la décomposition du carbalcoxydithiocarbamate intermédiaire de formule

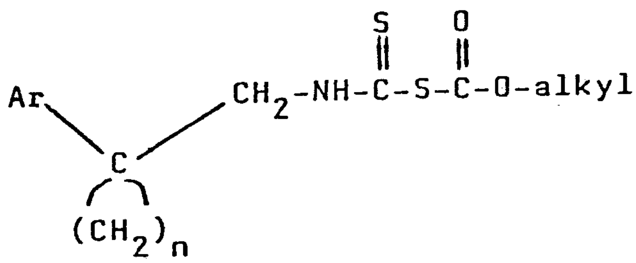

(VI)

5. Composition pour combattre les champignons, les bactéries et les insectes comprenant un véhicule inerte et, comme ingrédient actif, une quantité efficace d'un (1-aryl(ou hétérocyclique)cycloalkylméthyl)isothiocyanate ayant pour formule

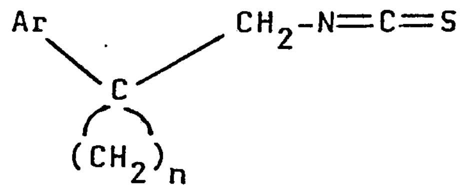

dans lequel

Ar est thiényle, halothiényle, naphtalényle, pyridinyle, phényle ou phényle substitué, ledit phényle substitué ayant de 1 à 3 substituants, chacun étant indépendamment choisi parmi un alcoyle de 1 à 6 atomes de carbone, un alcoyloxy de 1 à 6 atomes de carbone, un alkylthio de 1 à 6 atomes de carbone, halo, amino, nitro, cyano et trifluorométhyle; et

n est un nombre entier de 2 à 5 inclus.

6. Composition selon la revendication 5, où l'isothiocyanate est tel que revendiqué à la revendication 2 ou 3.

7. Composé selon l'une quelconque des revendications 1 à 3, ou composition selon l'une quelconque des revendications 5 ou 6 pour une utilisation comme agent fongicide, bactéricide ou insecticide.

**Revendications pour l'Etat contractant: AT**

1. Procédé de préparation d'un isothiocyanate de 1-aryl-(ou hétérocyclo)-cycloalcoylméthyle de formule:

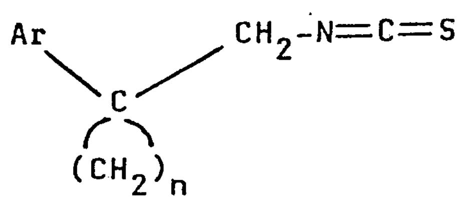

(I)

où Ar est un radical thiényle, halothiényle, naphtalényle, pyridinyle, phényle ou phényle substitué, lequel radical phényle substitué porte 1 à 3 substituants choisis chacun indépendamment parmi les radicaux alcoyle en $C_1$—$C_6$, alcoxy en $C_1$—$C_6$, alcoylthio en $C_1$—$C_6$, halo, amino, nitro, cyano et trifluorométhyle, et

n est un nombre entier de 2 à 5 inclusivement, caractérisé en ce qu'on fait réagir un composé de formule:

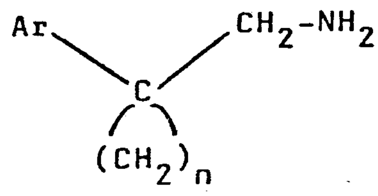

(II)

avec du disulfure de carbone (III) en présence d'hydroxyde de sodium pour former un composé de formule:

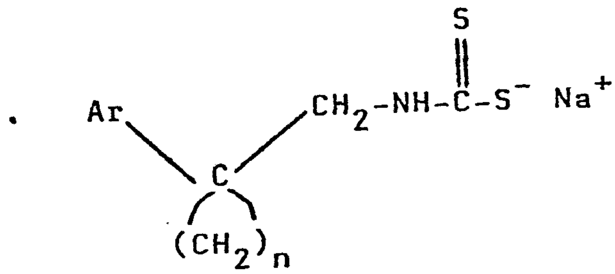

(IV)

puis on convertit ce dernier en l'isothiocyanate (I) désiré par réaction de (IV) avec un agent de décomposition consistant en un chloroformiate d'alcoyle en $C_1$—$C_6$, la réaction de l'amine (II) avec le disulfure de carbone (III) étant exécutée dans un solvant approprié inerte à l'égard de la réaction, la température de réaction étant maintenue inférieure à la température ambiante, et la réaction de décomposition du dithiocarbamate (IV) par sa réaction avec le chloroformiate d'alcoyle en $C_1$—$C_6$ (V) étant advantageusement conduite à des températures élevées dans un solvant approprié inerte à l'égard de la réaction, et une base étant ajoutée pour exercer un effet catalytique sur la décomposition du carbalcoxydithiocarbamate intermédiaire formé de formule:

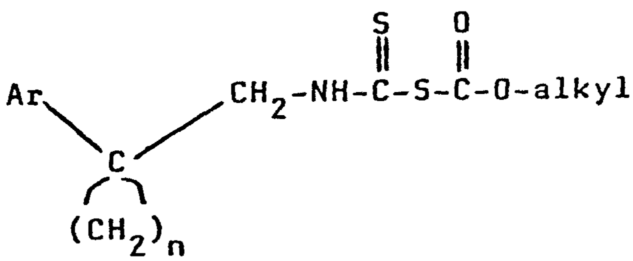

(VI)

2. Procédé suivant la revendication 1 de préparation de l'isothiocyanate de 1'phénylcyclopropylméthyle.

3. Procédé suivant la revendication 1 de préparation de l'isothiocyanate de 1-(2-thiényl)cyclopropylméthyle.

4. Procédé suivant l'une quelconque des revendications 1 à 3, caractérisé en ce que le chloroformiate d'alcoyle en $C_1$—$C_6$ (V) est le chloroformiate d'éthyle.

5. Procédé suivant l'une quelconque des revendications 1 à 4, caractérisé en ce que la base est un hydroxyde de métal alcalin ou de métal alcalinoterreux.

6. Composition pour combattre les champignons, bactéries et insectes, caractérisée en ce qu'elle comprend un excipient inerte et, comme constituant actif, une quantité efficace d'un isothiocyanate de 1-aryl-(ou hétérocyclo)-cycloalcoylméthyle de formule:

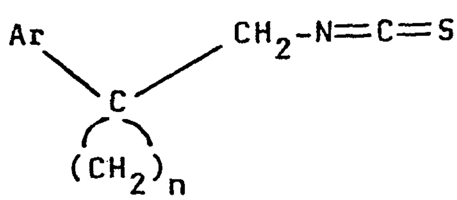

où Ar est un radical thiényle, halothiényle, naphtalényle, pyridinyle, phényle ou phényle substitué, lequel radical phényle substitué porte 1 à 3 substituants choisis chacun indépendamment parmi les radicaux alcoyle en $C_1$—$C_6$, alcoxy en $C_1$—$C_6$, alcoylthio en $C_1$—$C_6$, halo, amino, nitro, cyano et trifluorométhyle, et

n est un nombre entier de 2 à 5 inclusivement.

7. Composition suivant la revendication 6, caractérisée en ce que l'isothiocyanate est l'isothiocyanate de 1-phénylcyclopropylméthyle ou l'isothiocyanate de 1-(2-thiényl)cyclopropylméthyle.

**Patentansprüche für die Vertragsstaaten BE, CH, DE, FR, GB, IT, W, NL, SE**

1. (1-Aryl (oder -heterocyclisches)-cycloalkylmethyl)isothiocyanat der Formel

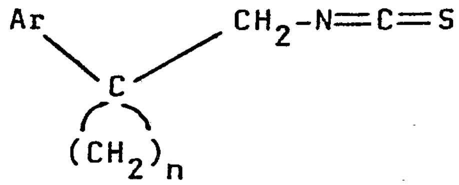

in der Ar eine Thienyl-, Halogenthienyl-, Naphthalenyl-, Pyridinyl-, Phenyl- oder 1 bis 3-fach substituierte Phenylgruppe bedeutet, wobei die Substituenten an der Phenylgruppe gleich oder verschieden sind und einen $C_1$—$C_6$-Alkyl-, $C_1$—$C_6$-Alkyloxy oder $C_1$—$C_6$-Alkylthiorest, ein Halogenatom oder eine Amino-, Nitro-, Cyano- oder Trifluormethylgruppe darstellen, und

n eine ganze Zahl von 2 bis einschließlich 5 ist.

2. (1-Phenylcyclopropylmethyl)-isothiocyanat.

3. [1-(2-Thienyl)-cyclopropylmethyl]-isothiocyanat.

4. Verfahren zur Herstellung eines (1-Aryl (oder -heterocyclischen) Cycloalkylmethyl)-isothiocyanats der Formel

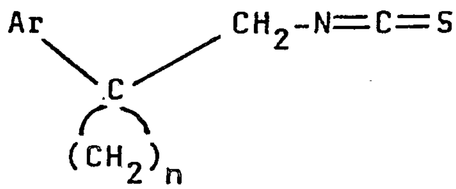

(I)

in der

Ar eine Thienyl-, Halogenthienyl-, Naphthalenyl-, Pyridinyl-, Phenyl- oder 1 bis 3-fach substituierte Phenylgruppe bedeutet, wobei die Substituenten an der Phenylgruppe gleich oder verschieden sind und einen $C_1$—$C_6$-Alkyl-, $C_1$—$C_6$-Alkyloxy- oder $C_1$—$C_6$-Alkylthiorest, ein Halogenatom oder eine Amino-, Nitro-, Cyano- oder Trifluormethylgruppe darstellen und

n eine ganze Zahl von 2 bis einschließlich 5 ist, dadurch gekennzeichnet, daß man eine Verbindung der Formel

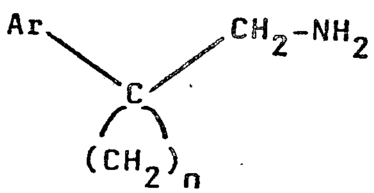

(II)

mit Schwefelkohlenstoff (III) in Gegenwart von Natriumhydroxid zu einer Verbindung der Formel

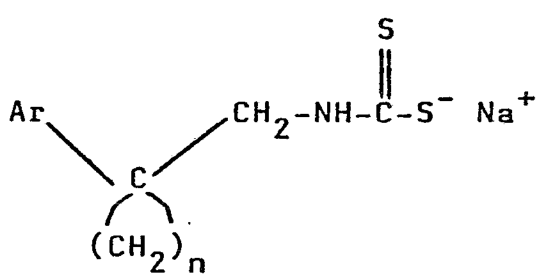

(IV)

umsetzt, anschließend die erhaltene Verbindung mit Hilfe eines Chlorameisensäure-$C_1$—$C_6$-alkylesters zersetzt und somit in das gewünschte Thiocyanat (I) überführt, wobei die Umsetzung des Amins (II) mit Schwefelkohlenstoff (III) in einem geeigneten inerten Lösungsmittel durchgeführt wird, die Reaktionstemperatur unterhalb Raumtemperatur gehalten wird und die mit Hilfe des Chlorameisensäure-$C_1$—$C_6$-alkylesters (V) erfolgende Zersetzung des Dithiocarbamats (IV) bei entsprechend erhöhter Temperatur

in einem geeigneten inerten Lösungsmittel durchgeführt wird und auf die Zersetzung des intermediär gebildeten Carbalkoxydithiocarbamats der Formel

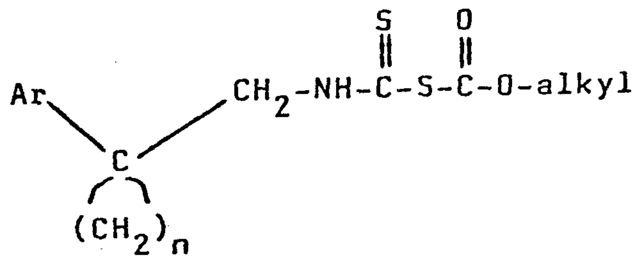

(VI)

durch Zusatz einer Base eine katalytische Wirkung ausgeübt wird.

5. Mittel zur Bekämpfung von pathogenen Pilzen, Bakterien und Insekten, enthaltend inertes Trägermaterial und, als aktiven Wirkstoff, eine effektive Menge eine (1-Aryl (oder -heterocyclischen)-cycloalkylmethyl)-isothiocyanats der Formel

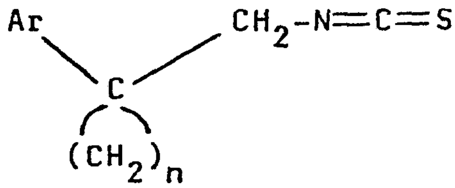

in der

Ar eine Thienyl-, Halogenthienyl-, Naphthalenyl-, Pyridinyl-, Phenyl- oder 1 bis 3-fach substituierte Phenylgruppe bedeutet, wobei die Substituenten an der Phenylgruppe gleich oder verschieden sind und einen $C_1$—$C_6$-Alkyl-, $C_1$—$C_6$-Alkyloxy- oder $C_1$—$C_6$-Alkylthiorest, ein Halogenatom oder eine Amino-, Nitro-, Cyano- oder Trifluormethylgruppe darstellen, und

n eine ganze Zahl von 2 bis einschließlich 5 ist.

6. Mittel nach Anspruch 5, wobei das Isothiocyanat eine Verbindung gemäß Anspruch 2 oder 3 ist.

7. Verbindung nach einem der Ansprüche 1 bis 3 oder ein Mittel nach Anspruch 5 oder 6, zur Verwendung als Fungizid, Bakterizid oder Insektizid.

**Patentansprüche für den Vertragsstaat: AT**

1. Verfahren zur Herstellung eines (1-Aryl- (oder Heterocyclyl) -cycloalkylmethyl)-isothiocyanats der Formel

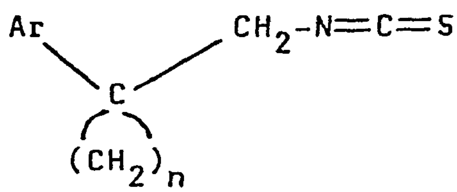

, (I)

worin

Ar Thienyl, Halogenthienyl, Naphthalenyl, Pyridinyl, Phenyl oder substituiertes Phenyl bedeutet, wobei das genannte substituierte Phenyl 1 bis 3 Substituenten aufweist, die unabhängig voneinander unter $C_1$—$C_6$-Alkyl, $C_1$—$C_6$-Alkyloxy, $C_1$—$C_6$-Alkylthio, Halogen, Amino, Nitro, Cyano und Trifluormethyl ausgewählt sind; und

n eine ganze Zahl von 2 bis einschließlich 5 bedeutet; dadurch gekennzeichnet, daß man eine Verbindung der Formel

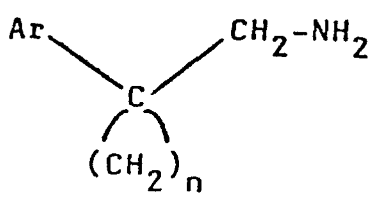

(II)

mit Schwefelkohlenstoff (III) in Gegenwart von Natriumhydroxid zur Herstellung einer Verbindung der Formel

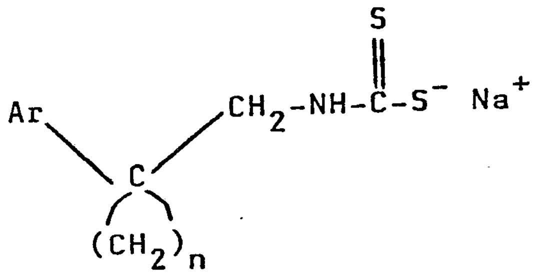

(IV)

umsetzt und anschließend die letztgenannte Verbindung in das gewünschte Isothiocyanat (I) durch Umsetzung von (IV) mit einem Zersetzungsmittel, bestehend aus einem ($C_1$—$C_6$-Alkylchlorformiat, umwandelt, wobei die Umsetzung des Amins (II) mit Schwefelkohlenstoff (III) in einem geeigneten reaktionsinerten Lösungsmittel ausgeführt wird und die Reaktionstemperatur unter Raumtemperatur gehalten wird, und die Zersetzungsreaktion des Dithiocarbamats (IV) durch dessen Reaktion mit dem $C_1$—$C_6$-Alkylchlorformiat (V) zweckmäßig bei erhöhten Temperaturen in einem geeigneten reaktionsinerten Lösungsmittel ausgeführt wird, und wobei eine Base zugesetzt wird, um einen katalytischen Effekt auf die Zersetzung des intermediär gebildeten Carbalkoxydithiocarbamats der Formel

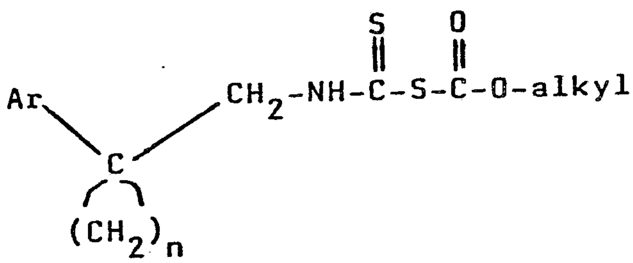

(VI)

zu ergeben.

2. Verfahren nach Anspruch 1 zur Herstellung von (1-Phenylcyclopropylmethyl)-isothiocyanat.

3. Verfahren nach Anspruch 1 zur Herstellung von [1-(2-Thienyl)-cyclopropylmethyl]-isothiocyanat.

4. Verfahren nach einem der Ansprüche 1 bis 3, dadurch gekennzeichnet, daß als $C_1$—$C_6$-Alkylchlorformiat (V) Ethylchlorformiat eingesetzt wird.

5. Verfahren nach einem der Ansprüche 1 bis 4, dadurch gekennzeichnet, daß als Base ein Alkalimetall- oder Erdalkalimetallhydroxid zugesetzt wird.

6. Zusammensetzung zur Bekämpfung von Pilzen, Bakterien und Insekten, umfassend ein inertes Trägermaterial und, als wirksamen Bestandteil, eine wirksame Menge eines (1-Aryl- (oder Heterocyclyl) -cycloalkylmethyl)-isothiocyanats der allgemeinen Formel

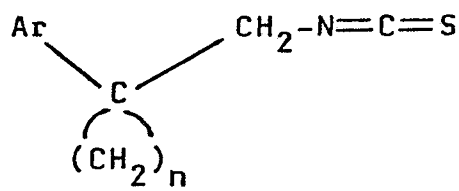

worin

Ar Thienyl, Halogenthienyl, Napthalenyl, Pyridinyl, Phenyl oder substituiertes Phenyl bedeutet, wobei das genannte substituierte Phenyl 1 bis 3 Substituenten aufweist, die unabhängig voneinander unter $C_1$—$C_6$-Alkyl, $C_1$—$C_6$-Alkyloxy, $C_1$—$C_6$-Alkylthio, Halogen, Amino, Nitro, Cyano und Trifluormethyl ausgewählt sind; und

n eine ganze Zahl von 2 bis einschließlich 5 bedeutet.

7. Zusammensetzung nach Anspruch 6, dadurch gekennzeichnet, daß sie als Isothiocyanat die in Anspruch 2 oder Anspruch 3 genannte Verbindung enthält.